# EUROPEAN PATENT APPLICATION

(11) **EP 4 837 894 A1**
(43) Date of publication of application: **11.11.2026**
(21) Application number: 25174771.3
(22) Date of filing: 07.05.2025
(51) Int. Cl.: C07B 41/04, C07B 41/12, C07C 67/00, C07C 67/287, C07C 69/734, C07C 253/20, C07D 213/30, C07D 307/92

(54) **A PHOTOMEDIATED SYNTHESIS METHOD FOR INTRODUCING A TRIFLUOROALKOXY MOIETY INTO AT LEAST ONE ORGANIC COMPOUND**

(71) Applicant: Freie Universität Berlin, 14195 Berlin (DE)
(72) Inventor: HASENSTAB-RIEDEL, Sebastian, 14532 Kleinmachnow (DE); KLEOFF, Merlin, 14165 Berlin (DE); CHRISTMANN, Mathias, 14532 Kleinmachnow (DE); SHAKERI, Kamar, 14109 Berlin (DE); GOLZ, Paul, 10779 Berlin (DE)
(74) Representative: Maikowski & Ninnemann Patentanwälte Partnerschaft mbB

(57) **Abstract**

The invention relates to a photomediated synthesis method for introducing a trifluoralkoxy moiety into at least one organic compound using a peroxide reagent in the presence of UV-light and optionally a catalyst.

## Description

The present invention relates to a photomediated synthesis method for introducing a trifluoroalkoxy moiety into at least one organic compound and compounds obtained by this method.

### Description

The introduction of fluorine into agrochemicals and pharmaceuticals has evolved as an indispensable tool to finetune their metabolic stability and lipohilicity and to influence their conformation. On the other hand, given the high stability of many fluorinated molecules, the EU is currently initiating a ban of per- and polyfluoroalkyl substances (PFAS). Excepted from this ban will be compounds with, e.g., trifluoromethoxy groups (-OCF₃) as they can degrade under biological conditions to non-persistent molecules.

Notably, among the approved fluoro-pharmaceuticals, 68% contain a C-F bond and 22% contain a C-CF₃ moiety, while only 2% bear a C-OCF₃ bond. This underlines that OCF₃-containing molecules are highly underrepresented despite their great potential. This has been mainly attributed to the lack of synthetically useful methods to prepare trifluoromethoxylated compounds. The direct introduction of a trifluoromethoxy group by C-H functionalization is particularly useful allowing a rapid synthesis of OCF₃-containing target molecules in drug research.

While there have been some reports for the C-H trifluoromethoxylation of aromatic compounds, the C-H trifluoromethoxylation of aliphatic compounds is highly underexplored. Only one method for the benzylic C-H trifluoromethoxylation has been developed by Xu, Tang, and coworkers using silver salts in catalytic to stoichiometric amounts depending on the substrate (Yang, H.; Wang, F.; Jiang, X.; Zhou, Y.; Xu, X; Tang, P. Silver-Promoted Oxidative Benzylic C-H Trifluoromethoxylation Angew. Chem. Int. Ed. 2018, 57, 13266-13270).

Moreover, no general method for the trifluoromethoxylation of non-activated alkanes has been reported, thus far, significantly limiting the access to trifluoromethoxylated alkyl substances.

Radical trifluoromethoxylation has been traditionally seldom conducted, however, in recent years the development of new radical OCF₃-transfer reagents has reinvigorated the field, opening up new synthetic routes towards trifluoromethoxylated molecules.

In 2018, the groups of Ngai and Togni, followed by Tang and co-workers in 2020, introduced bench stable reagents which release OCF₃ radicals upon photocatalytic activation of an N-O or S-O bond (see Scheme 1) (W. Zheng, C. A. Morales-Rivera, J. W. Lee, P. Liu, M.-Y. Ngai, Angew. Chem. Int. Ed. 2018, 57, 9645-9649; W. Zheng, J. W. Lee, C. A. Morales-Rivera, P. Liu, M.-Y. Ngai, Angew. Chem. Int. Ed. 2018, 57, 13795-13799.; B. J. Jelier, P. F. Tripet, E. Pietrasiak, I. Franzoni, G. Jeschke, A. Togni, Angew. Chem. Int. Ed. 2018, 57, 13784-13789; Z. Deng, M. Zhao, F. Wang, P. Tang, Nature Communications 2020, 11, 2569).

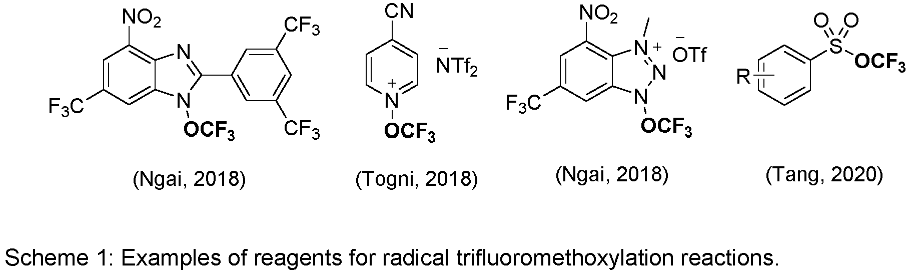

Given the expensive synthesis and low atom economy of these reagents, Dix et al. (S. Dix, P. Golz, J. R. Schmid, S. Riedel, M. N. Hopkinson, Chemistry 2021, 27, 11554-11558) recently developed bis(trifluoromethyl)-peroxide (BTMP) as a practical and comparatively inexpensive reagent for the direct trifluoromethoxylation of (hetero)arenes (Scheme 2).

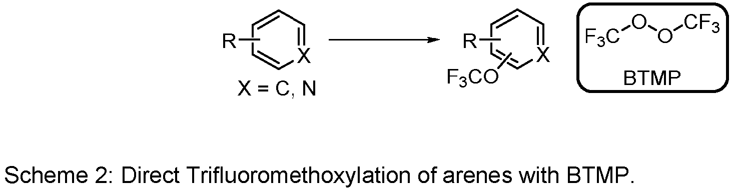

The groups of Hopkinson and Riedel demonstrated that bis(trifluoromethyl)peroxide (BTMP, (F₃CO)₂) can be used for the trifluoromethoxylation of arenes, silyl enol ethers, and allyl silanes (Hopkinson, M. N. Catalyst-Free Trifluoromethoxylation of Silyl Enol Ethers and Allyl Silanes with Bis(trifluoromethyl)peroxide Angew. Chem. Int. Ed. 2024, 63, e202317770).

Notably, BTMP, although being a peroxide, is remarkably stable up to 200 °C, is an easy-to-handle gas, and can be prepared in large quantities from abundant starting materials (Nissen, J. H.; Drews, T.; Schröder, B.; Beckers, H.; Steinhauer, S.; Riedel, S. Perfluoro Alkyl Hypofluorites and Peroxides Revisited Chem. Eur. J. 2019, 25, 14721-14727; Nissen, J. H.; Stücker, T.; Drews, T.; Steinhauer, S.; Beckers, H.; Riedel, S. No Fear of Perfluorinated Peroxides: Syntheses and Solid-State Structures of Surprisingly Inert Perfluoroalkyl Peroxides Angew. Chem. Int. Ed. 2019, 58, 3584-3588).

Nevertheless, the number of methods for the aliphatic trifluoromethoxylation remains small. In 2021, Duhail et al. (T. Duhail, T. Bortolato, J. Mateos, E. Anselmi, B. Jelier, A. Togni, E. Magnier, G. Dagousset, L. Dell'Amico, Organic Letters 2021, 23, 7088-7093) presented the direct synthesis of *α*-trifluoromethoxylated ketones using the Togni OCF₃ reagent under photoredox catalytic activation.

It is thus desirable to provide alternative methods for introducing fluorinated alkoxy moieties into organic compounds. It is of a particular interest to find alternative methods for the direct C-H trifluoralkoxylation, in particular trifluoromethoxylation in benzylic positions, of non-activated alkanes, and aldehydes.

Accordingly, a photomediated synthesis method for introducing a trifluoroalkoxy moiety into at least one organic compound is provided, wherein the method comprises the following steps:
- Providing at least one organic compound comprising at least one of the following general formulae (I) 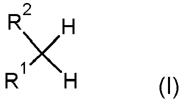
   with R¹ being selected from substituted or unsubstituted Aryl or substituted or unsubstituted Heteroaryl,
   with R² being selected from H, substituted or unsubstituted Alkyl, substituted or unsubstituted Aryl, substituted or unsubstituted Cycloalkyl and substituted or unsubstituted alkoxycarbonyl, or
- Providing at least one organic compound comprising at least one of the following general formulae (II) 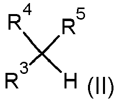
   with R³, R⁴, R⁵ being selected from substituted or unsubstituted alkyl, or being part of a monocyclic, a bicyclic ring system, or polycylic ring system, and
   wherein R³, R⁴ and R⁵ can be the same or different,
   with R₅ in addition being selected from H,
   wherein R³ and R⁴ can be the same or different, if R⁵ is H, or
- Providing at least one organic compound comprising at least one of the following general formulae (III) 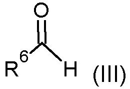 with R⁶ being selected from substituted or unsubstituted aryl or being selected from substituted or unsubstituted alkyl,
- Reacting the at least one organic compound of general formulae (I), (II), (III) with at least one peroxide reagent according to the general formulae (IV)

   (OCₘF₂ₘ₊₁)₂

   with C being ¹²C or ¹³C,
   with m being an integer in the range from 1 to 4, preferably from 1 to 3, most preferably 1 in the presence of at least one organic solvent, and optionally at least one catalyst by irradiating with UV light, and
- Obtaining at least one organic compound comprising at least one of the following general formulae (V), (VI) or (VII) with at least one trifluoroalkoxy moiety 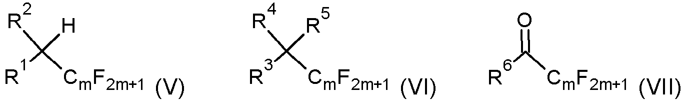
wherein the substituents R¹, R², R³, R⁴, R⁵ and R⁶ have the meanings as described above for general formulae (I), (II) and (III).

Thus, a method is provided that allows for direct C-H trifluoralkoxylation, in particular trifluormethoxylation, for functionalizing benzylic, non-activated, and aldehydic positions.

In addition, the method allows for preparation of stable-isotope labeled substrates using a¹³C-labeled peroxide reagent of general formulae (IV). The ¹³C-labeled substrates hold immense potential in the detection of drugs within biological systems allowing improved tracking of metabolic processes thereby accelerating drug discovery.

The method is carried out in a suitable organic solvent. The at least one organic solvent may be selected from a group containing dichloromethane (DCM), Acetone, acetonitrile (MeCN), Nitromethane, Dimethyl sulphoxide (DMSO), Ethyl acetate (ETAc), and water (H₂O) or a mixture thereof. The preferred organic solvent is acetone or an aqueous mixture thereof.

In an embodiment, the method is carried out in the presence of at least one catalyst. The at least one catalyst preferably used in the present method, is a tungsten containing salt, in particular a decatungstate containing salt, more in particular an ammonium cation or sodium cation decatungstate containing salt, such as tetrabutylammonium decatungstate (TBADT).

The photomediated reaction is carried out at a temperature between 18°C and 25°C, preferably between 20°C and 23°C, such as room temperature. Thus, the method can be carried out at ambient temperatures.

As indicated above, the reaction mixture is irradiated with UV light, specifically at 365 nm.

In another embodiment, an alkali fluoride, such as KF, may be added to the reaction mixture. This has shown to be an in particular advantage in case of trifluoralkoxylation of non-activated C-H bonds as the yield increased by adding KF. It is believed that KF acts as type of HF scavenger by forming KHF₂ (potassium bifluoride).

In an embodiment of the present method, the starting compound of general formulae (I) comprises benzylic C-H bonds, wherein H is subsequently replaced by a trifluoroalkoxy group. The moieties R¹ and R² of general formulae (I) 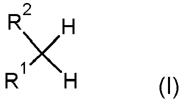 can be one of the following:
- R¹ being selected from substituted or unsubstituted - C₆H₅, preferably -C₆H₅,
- C₆H₄-Hal, preferably -C₆H₄-F, -C₆H₄-CN, -C₆H₄-CF₃,
- C₆H₄-alkyl, preferably -C₆H₄-CH₃, -C₆H₄-C(CH₃)₃,
- C₆H₄-C₆ aryl, preferably -C₆H₄-C₆H₅,
- C₆H₄-C₆H₄-Hal, preferably -C₆H₄-C₆H₄-F, -C₆H₄-C₆H₄-CN, -C₆H₄-C₆H₄-CF₃,
- C₆H₄₋₋C₆H₄-alkyl, preferably -C₆H₄-C₆H₄-CH₃, -C₆H₄-C₆H₄-CH(CH₃)₂, -C₆H₄-C₆H₄-C(CH₃)₃,
- C₆H₄-heteroaryl, preferably -C₆H₄-C₅H₄N; or
   R¹ being selected from substituted or unsubstituted - C₄-C₅ Heteroaryl, in particular C₄-C₅₋ S, O, N Heteroaryl, such as pyridine, pyrimidine (1,3-dazine), pyridazine (1,2-diazine), pyrazine (1,4 diazine), furan, thiophene-S-oxide, thiopene-S,S-dioxide, and
- R² being selected from substituted or unsubstituted C₁-C₆ alkyl, preferably -CH₃, - CH(CH₃)₂, -C(CH₃)₃, substituted or unsubstituted C₁-C₆ alkoxycarbonyl, preferably - (CH₂)ₙOCOR' with n being 1-6, preferably 1-4, R' being C₁-C₆ alkyl, preferably -CH₃, -C₆H₅, - C₆H₄-Hal, such as -C₆H₄-Br, substituted or unsubstituted C₄-C₁₀ cycloalkyl, substituted or unsubstituted C₆ aryl.

In another embodiment, moieties R¹ and R² of general formulae (I) can be one of the following:
- R¹: 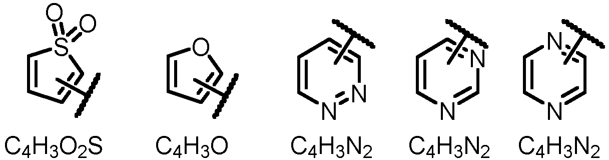
- R²: 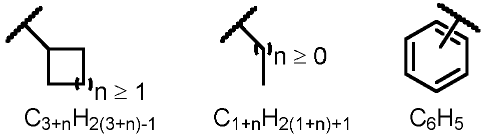
with n being 1-6, preferably 1-4.

In a more preferred embodiment, moieties R¹ and R² of general formulae (I) can be one of the following:
- R¹ being selected from
- C₆H₅, -C₆H₄-C₆H₅, -C₆H₄-F, -C₆H₄-CN, -C₆H₄-CF₃, -C₆H₄-CH₃, -C₆H₄-C(CH₃)₃,
- C₆H₄-C₆H₄-F, -C₆H₄-C₆H₄-CN, -C₆H₄-C₆H₄-CF₃, -C₆H₄-C₆H₄-CH₃, -C₆H₄-C₆H₄-CH(CH₃)₂ ,-C₆H₄-C₆H₄-C(CH₃)₃,
- C₆H₄-C₅H₄N; or
- R² being selected from -CH₃, -CH(CH₃)₂, -C(CH₃)₃, -(CH₂)ₙOCOR' with n being 1-4, R' being -CH₃, -C₆H₅, -C₆H₄-Br.

When using a compound of general formulae (I) as starting material, the present method can be conducted in the presence of the above described catalyst or without any catalyst (i.e. only be irradiating with UV-light).

In an embodiment of the present method, the starting compound of general formulae (II) comprises non-activated C-H bonds, wherein H is subsequently replaced by a trifluoroalkoxy group. The moieties R³, R⁴, R⁵ of general formulae (II) 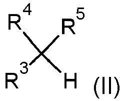 can be one of the following:
- R³, R⁴, R⁵ being selected from substituted or unsubstituted C₁-C₆ alkyl or being part of a monocyclic C₆-C₁₂ cycloalkyl, a -C₆-C₁₂ cycloalkyl fused with lactones, a diamantoide ring systems, such as adamantan, diamantan, wherein R³, R⁴ and R⁵ can be the same or different, and with R₅ in addition being selected from H, wherein R³ and R⁴ can be the same or different, if R⁵ is H.

Thus, the compound of general formulae (II) can be provided as secondary compound if R⁵ is H or as a tertiary compound if R⁵ is not H; i.e. the trifluoroalkoxy moiety can be introduced at a secondary or tertiary C atom of compound (II).

In a more preferred embodiment, moieties R³, R⁴, R⁵ of general formulae (II) can be one of the following:
- R³, R⁴ being moieties as part of a monocyclic C₆ or C₁₂ cycloalkyl, a C₆ or C₁₂ cycloalkyl fused with a lacton, with R₅ being H, or
- R³, R⁴, R⁵ being moieties as part of an adamantan system or a diamantan system.

When using a compound of general formulae (II) as starting material, the present method is preferably conducted in the presence of the above described catalyst and UV-light.

In an embodiment of the present method, the starting compound of general formulae (III) comprises aldehydic C-H bonds, wherein H is subsequently replaced by a trifluoroalkoxy group. The moiety R⁵ of general formulae (III) 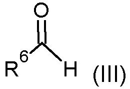 can be one of the following:
- R⁶ being selected from substituted or unsubstituted C₆-C₁₂ aryl, preferably - C₆H₅, -C₆H₄-C₆ aryl, -C₆H₄-Hal, preferably -C₆H₄-F, -C₆H₄-CN, -C₆H₄-CF₃, - C₆H₄-alkyl, -C₆H₄-C₁-C₆ alkyl, such as -C₆H₄-CH₃, -C₆H₄-C(CH₃)₃, or being selected from substituted or unsubstituted -C₁-C₁₀ alkyl, such as -C₂-C₈ alkyl, -C₂-C₈ alkyl - aryl, such as -C₇H₁₅ -C₂H₄-C₆ aryl.

In a preferred embodiment, moiety R⁶ of general formulae (III) can be one of the following:
- R⁶ being selected from -C₆H₅, -C₆H₅-C₆H₅, -C₆H₄-F, -C₆H₄-CN, -C₆H₄-CF₃, - C₆H₄-CH₃, -C₆H₄-C(CH₃)₃, or being selected from substituted or unsubstituted -C₂-C₈ alkyl, such as -C₇H₁₅ or -C₂-C₈ alkyl -aryl, such as -C₂H₄-C₆H₅.

When using a compound of general formulae (II) as starting material, the present method is preferably conducted in the presence of the above described catalyst and UV-light.

As indicated, any of the moieties mentioned above can be non-substituted or substituted. Here the term "substituted", in particular in connection to alkyl, cycloalkyl, aryl relates to the substitution of one or more atoms, usually H-atoms, by one or more of the following substituents: halogen, , protected hydroxy, oxo, C₁-C₁₅ alkyl, C₃-C₁₀-cycloalkyl, aryl, heteroaryl, naphthyl, imido, isocyano, protected amino, , heterocyclic ring, carbonate, imidazolyl, indolyl, C₁-C₁₂-acyl, C₁-C₁₂-acyloxy, nitro, nitroso, ester, aldehyde, ketone, N-(C₁-C₁₂-alkyl)carboxamide, C₁-C₁₂-(per)fluoroalkyl and C₁-C₁₀-alkylsulfonyl.

It is in preferred if any of the moieties mentioned above can be non-substituted or substituted with halogen, protected hydroxy, oxo, C₁-C₁₅ alkyl, C₃-C₁₀-cycloalkyl, aryl, heteroaryl, naphthyl, imido, protected amino,

It is in particular preferred if any of the moieties mentioned above can be non-substituted or substituted with halogen, , C₁-C₁₀ alkyl, such as methyl, ethyl or propyl, C₃-C₁₀-cycloalkyl,

The substituted groups can be once or twice substituted with same or different substituents. The alkyl moieties may also comprise one or multiple double bonds.

As described above, the present method requires at least one peroxide reagent according to the general formulae (IV)

(OCₘF₂ₘ₊₁)₂

with C being ¹²C or ¹³C, and
with m being an integer in the range from 1 to 4, preferably from 1 to 3, most preferably 1.

In an embodiment, the peroxide reagent according to the general formulae (IV) is selected from one of the following (CF₃O)₂, (C₂F₅O)₂, (C₃F₇O)₂ (linear or branched) or (C₄F₉O)₂ (linear or branched), wherein C is ¹²C or ¹³C.

The most preferred peroxide reagents are (¹²CF₃O)₂ (known as BTMP) or (¹³CF₃O)₂ (known as ¹³C-BTMP).

Accordingly, in a preferred embodiment of the present method, the organic compound of general formulae (I) 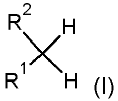 is reacted with Bis(trifluoromethyl)-peroxide (BTMP or ¹³C-BTMP) in the presence of at least one organic solvent, and optionally at least one catalyst, such as tetrabutylammonium decatungstate (TBADT), by irradiating with UV light providing an organic compound of general formulae (Va) with one trifluoromethoxy moiety 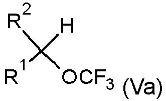 with R¹ and R² having the above described meanings.

In preferred embodiments the organic compound of general formulae (Va) obtained by the present method may be one of the following compounds:

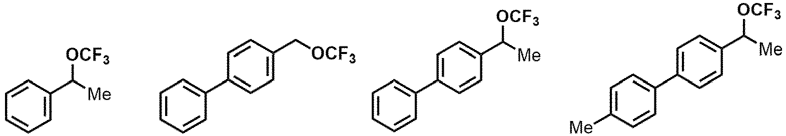

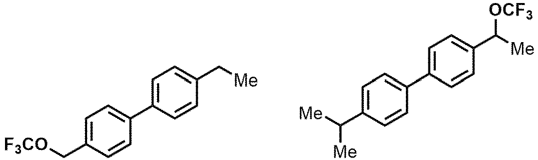

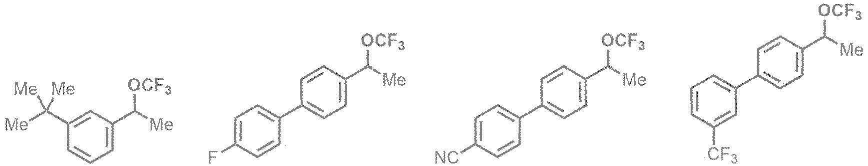

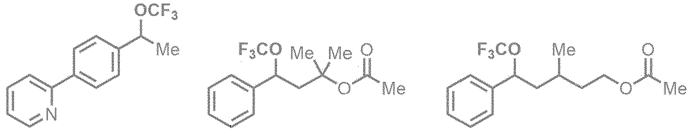

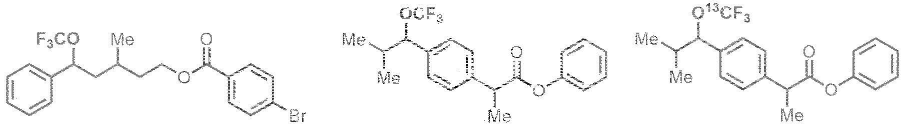

In another preferred embodiment of the present method, the organic compound of general formulae (II) 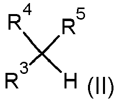 is reacted with Bis(trifluoromethyl)-peroxide (BTMP or ¹³C-BTMP) in the presence of at least one organic solvent, and optionally at least one catalyst, such as tetrabutylammonium decatungstate (TBADT), by irradiating with UV light providing an organic compound general formulae (Vla) with one trifluoromethoxy moiety 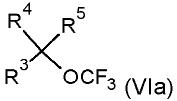 with R³, R⁴, R⁵ having the above described meanings.

In preferred embodiments the organic compound of general formulae (Vla) obtained by the present method may be one of the following compounds:

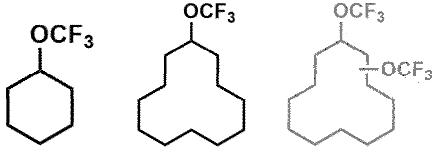

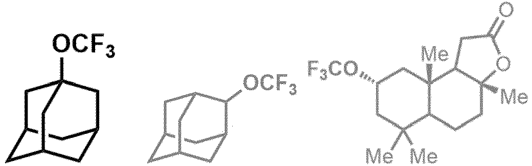

In another preferred embodiment of the present method, the organic compound of general formulae (III) 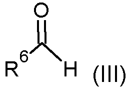 is reacted with Bis(trifluoromethyl)-peroxide (BTMP or ¹³C-BTMP) in the presence of at least one organic solvent, and optionally at least one catalyst, such as tetrabutylammonium decatungstate (TBADT), by irradiating with UV light providing an organic compound general formulae (Vlla) with one trifluoromethoxy moiety 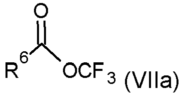 with R⁶ having the above described meanings.

In preferred embodiments the organic compound of general formulae (Vlla) obtained by the present method may be one of the following compounds:

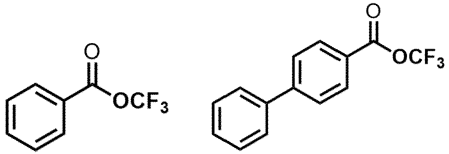

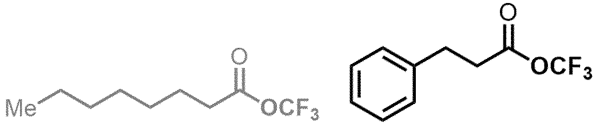

The invention is explained in more detail by means of the following figures and examples. It shows:
- Figure 1: an overview of compounds synthesized by the method according to the invention; and
- Figure 2: a reaction scheme for ¹³C labelling using the method according to the invention.

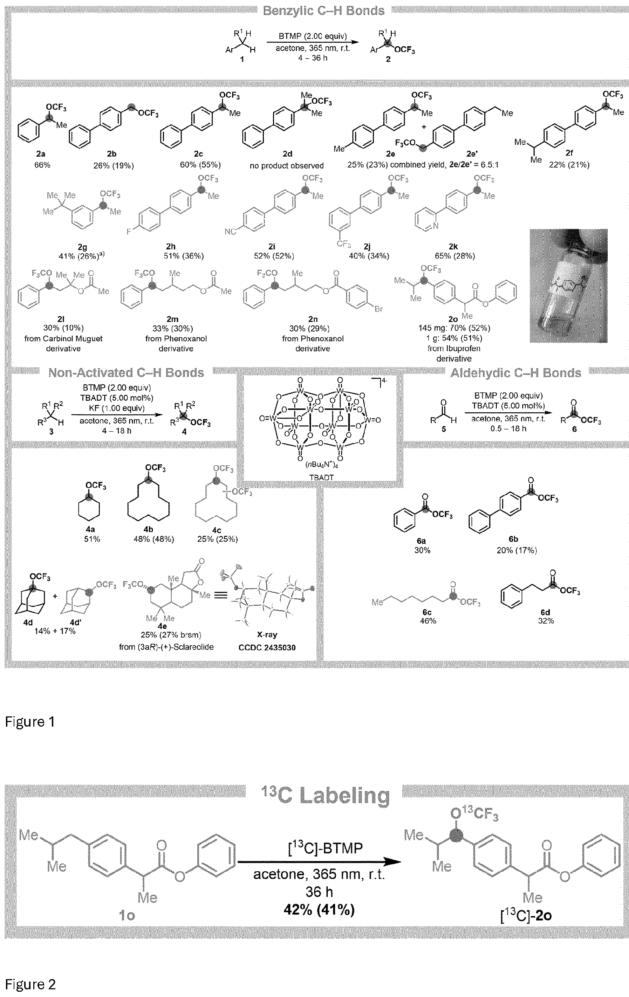

As illustrated in the reaction scope of Figure 1, the present method allows the trifluormethoxylation of benzylic C-H bonds, non-activated C-H bonds and aldehydic C-H bonds.

At the outset, the C-H trifluoromethoxylation of benzylic positions with BTMP using ethylbenzene (1a) as screening system was investigated by using tetrabutylammonium decatungstate (TBADT) as catalyst to activate the benzylic C-H bonds. Reaction with BTMP provides the corresponding trifluoromethylether **2a.** Irradiation of a solution of ethylbenzene (**1a**) and TBADT in acetonitrile for 18 h gave **2a** in a yield of 18% (table 1, entry 1). Using acetone gave the desired product **2a** in significantly higher yield of 55% (table 1, entry 2).

**Table 1. Optimization and Control Experiments.**

| 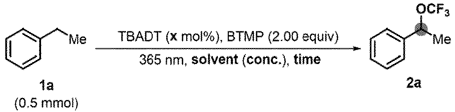 | | | | | |
|---|---|---|---|---|---|
| **entry** | **x (mol%)** | **solvent** | **conc**. **(M)** | **time [h]** | **yield^{a)} (%)** |
| 1 | 5 | MeCN | 0.24 | 18 | 18 |
| 2 | 5 | acetone | 0.24 | 18 | 55 |
| 3 | 5 | acetone/HFIP (10:1) | 0.24 | 18 | 34 |
| 4^{b)} | 5 | acetone | 0.24 | 18 | 29 |
| 5 | 5 | acetone | 0.48 | 18 | 46 |
| 6 | 10 | acetone | 0.24 | 18 | 31 |
| 7 | 5 | acetone | 0.24 | 2 | 60 |
| 8 | 5^{c)} | acetone | 0.24 | 2 | 27 |
| 9^{d)} | 5 | acetone | 0.24 | 4 | traces |
| 10 | - | **acetone** | **0.24** | **4** | **66** |

| | | | | | |
|---|---|---|---|---|---|
| ^{a)} Determined by ¹⁹F NMR spectroscopy using α,α,α-trifluorotoluene (PhCF₃) as internal standard. ^{b)} Performed with 1.00 equiv KF as additive. ^{c)} Using NaDT instead of TBADT. ^{d)} Reaction conducted under exclusion of light. | | | | | |

When adding hexafluoro iso-propanol (HFIP) the yield dropped to 34% (table 1, entry 3). Employing KF as additive did not have a positive impact on the reaction outcome (table 1, entry 4). Changing the molarity or catalyst loading did also result in diminished reaction yields (table 1, entry 5 and 6). When the reaction time was reduced to 2 h the yield increased to 60% (table 1, entry 7). Switching the catalyst to sodium decatungstate (NaDT) decreased the yield as well (table 1, entry 8). When performing control experiments under exclusion of light, only traces of 2a could be observed (table 1, entry 9). Surprisingly, in the absence of a catalyst, the yield increased even to 66% (table 1, entry 10).

Subsequently, the trifluoromethoxylation protocol was applied to a variety of benzylic substrates (Figure 1). First, the influence of primary, secondary and tertiary benzylic positions was investigated. In case of a primary position the corresponding trifluoromethoxy derivative **2b** was obtained in 19% yield. Changing to secondary benzylic carbon improved the yield significantly to 55% in case of **2c.** The protocol tolerated a couple of functional groups such as *tert*-butyl (**2e,** 26% yield), fluorine (**2f,** 36% yield), cyano (**2g,** 52% yield) and CF₃ (**2h,** 34% yield) groups. Also, a pyridinyl substituent was tolerated affording **2i** in 28% yield. Aliphatic and benzylic esters were also untouched under the reaction conditions as shown for the trifluoromethoxylated products of the odorants Carbinol Muguet **2j** (10% yield) and Phenoxanol **2k** (30% yield) and **2l** (29% yield). Notably, Ibuprofen derivative **1m** was selectively functionalized in the secondary benzylic position providing trifluoromethylether **2m** in 52% yield. To demonstrate the scalability of the trifluoromethoxylation protocol, **2m** was also prepared on gram scale in a similar yield of 51%.

Notably, the benzylic trifluoromethoxylation using BTMP as reagent offers an advantageous atom economy. In the case of compound **2c,** a 2:1 ratio of total mass of reagents to substrate is required using BTMP (55% yield), compared to a 15:1 ratio employing the previously reported method of Xu, Tang, and coworkers (58% yield).

Next, non-activated and aldehydic C-H bonds were functionalized. When using TBADT and KF in acetone trifluoromethylcyclohexyl ether **(4a)** in 51% ¹⁹F NMR yield was obtained. When using cyclododecane, **4b** could be isolated as colorless liquid (48% yield), which could be functionalized a second time under the same reaction conditions to yield double functionalized product **4c** (25% yield). Also, adamantane was C-H trifluoromethoxylated in the secondary and the tertiary positions giving **4d** and **4e** in 14% and 17% ¹⁹F NMR yield, respectively.

Notably, the protocol could also be adopted to the natural product (3aR)-(+)-sclareolide affording the corresponding trifluoromethylether **4f** as a single diastereomer in 25% yield. The structure of **4f** was unambiguously confirmed by single-crystal X-ray diffraction of crystals of **4f** obtained by slow evaporation of a solution of **4f** in a mixture of dichloromethane and cyclohexane.

When expanding the scope of C-H activated groups to aldehydes, it was possible to prepare the corresponding trifluoromethylesters **6a -6d.** In case of benzaldehyde **(5a)** the corresponding ester was obtained in 30% ¹⁹F NMR yield. Using the heavier analogue **5b,** the corresponding trifluoromethyl ester **6b** could be isolated in 17% yield. Also, aliphatic aldehydes were transformed as shown for **6c** (46% ¹⁹F NMR yield) and **6d** (32% ¹⁹F NMR yield).

For chemical, biomedical and environmental research, the preparation of stable-isotope labeled substrates is highly important. These substrates hold immense potential in the detection of drugs within biological systems allowing improved tracking of metabolic processes thereby accelerating drug discovery.

Therefore, a synthesis of the ¹³C-labeled bis(trifluoromethyl)peroxide ([¹³C]-BTMP) starting from commercially available ¹³CO and F₂ was developed. This reagent enables C-H trifluoromethoxylation and ¹³C-labeling of various compounds in a single step. Using [¹³C]-BTMP, this was shown as a representative example for the preparation of the [¹³C]-labelled Ibuprofen derivative [¹³C]-**2m** in 41% yield (see Figure 2).

### General Procedure for Trifluoromethoxylation of Benzylic C-H bonds (GP1)

A RETTBERG Schlenk tube was charged with a stir bar and a solution of the substrate (1.00 equiv) in acetone (0.24 M). The suspension was degassed by Freeze-Pump-Thaw procedure (2x). Then, BTMP (2.00 equiv) was condensed into the cooled (liquid nitrogen) solution, the flask was closed and the solution was allowed to melt and warm up to r.t. The reaction mixture was irradiated with UV-light (365 nm, 30 W LED Chip) for 2-36 h. The flask was opened slowly to release the overpressure. Then, the solvent was directly evaporated under reduced pressure and the product was purified by flash column chromatography.

### Example: 4-(1-(Trifluoromethoxy)ethyl)-1,1'-biphenyl

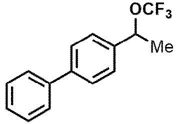

4-(1-(Trifluoromethoxy)ethyl-1,1'-biphenyl was prepared from 4-Ethyl-1,1'-biphenyl (91.0 mg, 0.500 mmol, 1.00 equiv) according to GP1 with a reaction time of 4 h. Purified by flash column chromatography (SiO2, pentane). 4-(1-(Trifluoromethoxy)ethyl-1,1'-biphenyl (73.0 mg, 0.270 mmol, 55% (60% crude)) was obtained as colorless solid.

1H NMR (500 MHz, CDCl3): δ = 7.63 - 7.58 (m, 4H), 7.48 - 7.42 (m, 4H), 7.39 - 7.35 (m, 1H), 5.36 (q, J = 6.6 Hz, 1H), 1.68 (d, J = 6.6 Hz, 3H) ppm.

13C{1H} NMR (151 MHz, CDCl3): δ = 141.8, 140.9, 139.7, 129.2, 127.9, 127.7, 127.5, 126.6, 122.1 (q, J = 255.3 Hz), 77.3, 23.7 ppm.

19F NMR (565 MHz, CDCl3): δ = -57.8 ppm.

The spectroscopic data fits to those reported in literature. (Yang, H.; Wang, F.; Jiang, X.; Zhou, Y.; Xu, X; Tang, P. Silver-Promoted Oxidative Benzylic C-H Trifluoromethoxylation Angew. Chem. Int. Ed. 2018, 57, 13266-13270).

### General Procedure for Trifluoromethoxylation of non-activated C-H bonds (GP2)

A RETTBERG Schlenk tube was charged with a stir bar, TBADT (5 mol%), KF (1.00 equiv), and a solution of the substrate (1.00 equiv) in acetone (0.48 M). The suspension was degassed by Freeze-Pump-Thaw procedure (2x). Then, BTMP (2.00 equiv) was condensed into the cooled (liquid nitrogen) solution, the flask was closed and the solution was allowed to melt and warm up to r.t. The reaction mixture was irradiated with UV-light (365 nm, 30 W LED Chip) for 2-18 h. The flask was opened slowly to release the overpressure. Then, the solvent was directly evaporated under reduced pressure and the product was purified by flash column chromatography.

### Example: (3aR,8S,9aS)-3a,6,6,9a-Tetramethyl-8-(trifluoromethoxy)decahydronaphtho[2,1-b]furan-2(1H)-one

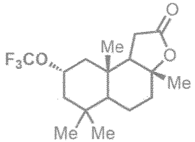

(3aR,8S,9aS)-3a,6,6,9a-Tetramethyl-8-(trifluoromethoxy)decahydronaphtho[2,1-b]furan-(1H)-one was prepared from (3aR)-(+)-sclareolide (250 mg, 1.00 mmol, 1.00 equiv) according to GP2 with a reaction time of 2 h. Purified by flash column chromatography (SiO2, CyH/EtOAc, 5:1). (3aR,8S,9aS)-3a,6,6,9a-Tetra-methyl-8-(trifluoromethoxy)decahydronaphtho [2,1-b]furan-2(1H)-one (64.0 mg, 0.200 mmol, 20% (25% crude), 27% brsm) was obtained as a colorless to light yellowish solid.

1H NMR (700 MHz, CDCl3): δ = 4.47 (tt, J = 11.5, 4.6 Hz, 1H), 2.45 (dd, J = 16.2, 14.7 Hz, 1H), 2.28 (dd, J = 16.1, 6.5, 0.5 Hz, 1H), 2.11 (dt, J = 12.0, 3.3 Hz, 1H), 2.02 (dd, J = 14.7, 6.5 Hz, 1H), 1.96 - 1.89 (m, 3H), 1.71 (td, J = 13.1, 12.6, 4.4 Hz, 1H), 1.41 - 1.36 (m, 2H), 1.34 (d, J = 1.0 Hz, 3H), 1.23 (t, J = 11.8 Hz, 1H), 1.12 (dd, J = 12.7, 2.8 Hz, 1H), 0.99 (s, 3H), 0.98 (s, 3H), 0.92 (s, 3H) ppm.

13C{1H} NMR (176 MHz, CDCl3): δ = 176.3, 122.0 (q, J = 254.4 Hz), 109.7, 86.1, 73.3, 59.0, 56.1, 48.1, 45.6, 38.7, 37.56, 35.1, 33.4, 29.0, 22.0, 20.5, 16.4 ppm.

19F NMR (376 MHz, CDCl3): δ = -57.7 ppm.

HRMS (EI): m/z calculated for [C17H25F3O3]+ ([M]+): 334.1756, measured: 334.1751.

### General Procedure for Trifluoromethoxylation of aldehydic C-H bonds (GP3)

A RETTBERG Schlenk tube was charged with a stir bar, TBADT (5 mol%), and a solution of the substrate (1.00 equiv) in acetone (0.24 M). The suspension was degassed by Freeze-Pump-Thaw procedure (2x). Then, BTMP (2.00 equiv) was condensed into the cooled (liquid nitrogen) solution, the flask was closed and the solution was allowed to melt and warm up to r.t. The reaction mixture was irradiated with UV-light (365 nm, 30 W LED Chip) for 0.5-18 h. The flask was opened slowly to release the overpressure. Then, the solvent was directly evaporated and the product was purified by flash column chromatography.

### Example: Trifluoromethyl [1,1'-biphenyl]-4-carboxylate

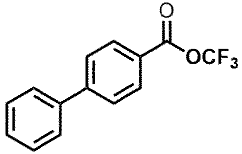

Trifluoromethyl [1,1'-biphenyl]-4-carboxylate was prepared from [1,1'-biphenyl]-4-carbaldehyde according to GP3 with a reaction time of 30 min. Purified by flash chromatography (SiO2, pentane/Et2O, 30:1 → 10:1). Trifluoromethyl [1,1'-biphenyl]-4-carboxylate (45.0 mg, 0.170 mmol, 17%, 62% brsm) was obtained as colorless solid.

1H NMR (600 MHz, CDCl3): δ = 8.16 - 8.12 (m, 2H), 7.75 - 7.72 (m, 2H), 7.65 (dd, J = 8.2, 1.1 Hz, 2H), 7.53 - 7.49 (m, 2H), 7.47 - 7.43 (m, 1H) ppm.

13C{1H} NMR (151 MHz, CDCl3): δ = 159.3, 148.2, 139.6, 131.5, 129.4, 129.1, 127.9, 127.7, 125.5, 120.3 (q, J = 265.4 Hz) ppm.

19F NMR (565 MHz, CDCl3): δ = -56.8 ppm.

The spectroscopic data fits to those reported in literature. (Zhu, H.; Gao, C.; Yu, T.; Xu, C.; Wang, M. O-Trifluoromethylation of Carboxylic Acids via the Formation and Activation of Acyloxy(phenyl)trifluoromethyl-λ3-lodanes Angew. Chem. Int. Ed. 2024, 63, e202400449.)

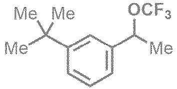

**¹H NMR** (600 MHz, CDCl₃): δ = 7.42 - 7.38 (m, 2H), 7.35 (t, *J* = 7.6 Hz, 1H), 7.21 (d, *J =* 7.3 Hz, 1H), 5.34 (q, *J* = 6.5 Hz, 1H), 1.68 (d, *J* = 6.6 Hz, 3H), 1.37 (s, 9H) ppm.

**¹³C{¹H} NMR** (151 MHz, CDCl₃): δ = 151.7, 140.1, 128.4, 125.5, 122.9, 122.8, 121.8 (q, *J =* 255 Hz), 77.7 (q, *J* = 2 Hz), 34.9, 31.4, 23.5 ppm.

¹⁹F **NMR** (565 MHz, CDCl₃): δ = -57.7 ppm.

**HRMS** (EI): m/z calculated for [C₁₃H₁₇F₃O]⁺ ([M+]⁺): 246.1226, measured: 246.1222.

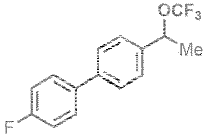

**¹H NMR** (700 MHz, CDCl₃): δ = 7.60 - 7.55 (m, 4H), 7.47 - 7.43 (m, 2H), 7.16 (t, *J* = 8.6 Hz, 2H), 5.38 (q, *J* = 6.6 Hz, 1H), 1.70 (d, *J* = 6.6 Hz, 3H) ppm.

**¹³C{¹H} NMR** (176 MHz, CDCl₃): δ = 162.9 (d, *J =* 247 Hz), 140.8, 139.8, 137.0 (d, *J =* 3.2 Hz), 129.0 (d, *J* = 8 Hz), 127.6, 126.6, 122.0 (q, *J* = 255 Hz), 116.1 (d, *J* = 21 Hz), 77.2, 23.7 ppm. **¹⁹F NMR** (376 MHz, CDCl₃): δ = -57.9, -115.2 - -115.3 (m) ppm.

**HRMS** (EI): m/z calculated for [C₁₅H₁₂F₄O]⁺([M]⁺): 284.0819, measured: 284.0828.

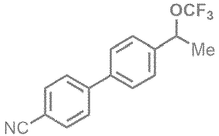

**¹H NMR** (400 MHz, CDCl₃): δ = 7.76 - 7.72 (m, 2H), 7.68 (d, *J* = 8.4 Hz, 2H), 7.63 - 7.58 (m, 2H), 7.47 (d, *J* = 8.3 Hz, 2H), 5.36 (q, *J* = 6.6 Hz, 1H), 1.67 (d, *J* = 6.5 Hz, 3H) ppm.

**¹³C{¹H} NMR** (176 MHz, CDCl₃): δ = 145.3, 141.3, 139.6, 133.0, 128.1, 127.9, 126.8, 122.0 (q, *J* = 255 Hz), 119.2, 111.6, 77.0 (q, *J* = 2 Hz), 23.7 ppm.

¹⁹F **NMR** (376 MHz, CDCl₃) δ = -58.0 ppm.

**HRMS** (EI): m/z calculated for [C₁₆H₁₂F₃NO]⁺([M]⁺): 291.0866, measured: 291.0853.

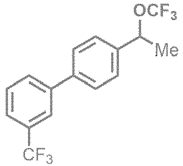

**¹H NMR** (400 MHz, CDCl₃): δ = 7.83 (s, 1H), 7.76 (d, *J* = 7.6 Hz, 1H), 7.62 (dd, J= 7.6, 5.5 Hz, 3H), 7.59 - 7.53 (m, 1H), 7.49 - 7.44 (m, 2H), 5.36 (q, *J* = 6.6 Hz, 1H), 1.68 (d, *J* = 6.5 Hz, 3H) ppm.

**¹³C{¹H} NMR** (176 MHz, CDCl₃): δ = 141.7, 140.7, 140.3, 131.6 (q, *J =* 32 Hz), 130.7, 129.7, 127.8, 126.8, 124.5 (q, 272 Hz), 124.5 (q, *J* = 4 Hz), 124.3 (q, *J* = 4 Hz), 122.0 (q, *J* = 255 Hz), 77.1 (q, *J* = 3 Hz), 23.7 ppm.

¹⁹F **NMR** (376 MHz, CDCl₃) δ = -57.9, -62.5 ppm.

**HRMS** (EI): m/z calculated for [C₁₆H₁₂F₆O]⁺([M]⁺): 334.0787, measured: 334.0790.

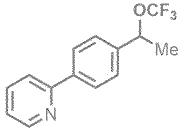

**¹H NMR** (600 MHz, CDCl₃): δ = 8.70 (d, *J* = 4.6 Hz, 1H), 8.01 (d, *J* = 8.3 Hz, 2H), 7.79 - 7.72 (m, 2H), 7.46 (d, *J* = 8.2 Hz, 2H), 7.26 - 7.23 (m, 1H), 5.36 (q, *J* = 6.6 Hz, 1H), 1.67 (d, *J =* 6.6 Hz, 3H) ppm.

**¹³C{¹H} NMR** (151 MHz, CDCL₃): δ = 157.2, 150.1, 141.5, 139.9, 137.2, 127.5, 126.5, 122.7, 122.0 (q, J = 255 Hz), 120.9, 77.3 - 77.2 (m), 23.7 ppm.

**¹⁹F NMR** (565 MHz, CDCl₃): δ = -57.9 ppm.

**HRMS** (EI): m/z calculated for [C₁₄H₁₂F₃NO]⁺([M]⁺): 267.0866, measured: 267.0870.

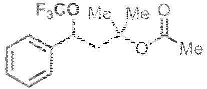

**¹H NMR** (700 MHz, CDCl₃): δ = 7.38 - 7.30 (m, 5H), 5.30 (dd, *J* = 8.5, 4.0 Hz, 1H), 2.54 (dd, *J* = 15.3, 8.5 Hz, 1H), 2.24 (dd, *J* = 15.2, 4.0 Hz, 1H), 1.89 (s, 3H), 1.55 (s, 3H), 1.49 (s, 3H) ppm.

**¹³C{¹H} NMR** (176 MHz, CDCl₃): δ = 170.8, 140.5, 129.0, 128.8, 126.5, 121.9 (q, *J =* 256 Hz), 80.5, 78.3 (q, *J* = 3 Hz), 47.3, 27.5, 26.9, 22.5 ppm.

**¹⁹F NMR** (376 MHz, CDCl₃): δ = -57.4 ppm.

**HRMS** (EI): m/z calculated for [C₁₂H₁₄F₃O]⁺ ([M-H₃CCO₂]⁺): 231.0991, measured: 231.0983.

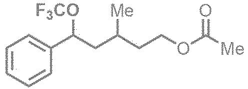

**¹H** NMR (600 MHz, CDCl₃): δ = 7.36 - 7.31 (m, 5H), 5.16 - 5.13 (m, 1H), 4.13 - 4.06 (m, 2H), 2.03 (s, 3H), 1.78 - 1.45 (m, 5H), 0.99 (d, *J* = 6.6 Hz, 3H) ppm.

**¹³C and ¹⁹F NMR data of major diastereomer:**

**¹³C{¹H}** NMR (151 MHz, CDCl₃): δ = 171.5, 140.3, 129.0, 128.8, 126.3, 123.7 (q, *J =* 256 Hz), 79.3 (q, *J* = 2.3 Hz), 62.6, 45.2, 35.8, 26.6, 21.3, 20.0 ppm.

**¹⁹F** NMR (564 MHz, CDCl₃): δ = -57.5 ppm.

**HRMS (EI):** m/z calculated for [C₁₅H₁₉F₃O₃]⁺ ([M+]⁺): 304.1281, measured: 304.1268.

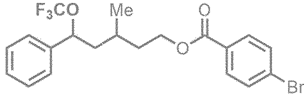

**¹H NMR** (400 MHz, CDCl₃): δ = 8.09 - 7.77 (m, 2H), 7.61 - 7.39 (m, 2H), 7.37 - 7.13 (m, 5H), 5.23 - 5.04 (m, 1H), 4.42 - 4.19 (m, 2H), 2.13 - 1.78 (m, 3H), 1.74 - 1.52 (m, 2H), 1.10 - 0.96 (m, 3H) ppm.

¹³C **NMR and ¹⁹F NMR data of one diastereomer:**

**¹³C{¹H} NMR** (151 MHz, CDCl₃): δ = 165.9, 140.0, 131.8, 131.7, 131.2, 128.8, 128.6, 121.8 (q, *J* = 256 Hz), 121.0, 79.1, 63.2, 45.0, 35.1, 26.4, 19.7 ppm.

**¹⁹F NMR** (376 MHz, CDCl₃): δ = -57.5 ppm.

**HRMS** (EI): m/z calculated for [C₂₀H₂₀BrF₃O₃]⁺([M]⁺): 444.0542, measured: 444.0539.

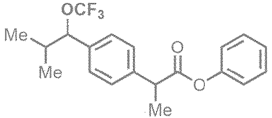

**¹H NMR** (600 MHz, CDCl₃): δ = 7.40 (d, *J* = 8.2 Hz, 2H), 7.34 (tt, *J* = 7.6, 2.2 Hz, 2H), 7.29 - 7.27 (m, 2H), 7.22 - 7.19 (m, 1H), 7.00 - 6.97 (m, 2H), 4.78 (d, *J* = 7.1 Hz, 1H), 3.98 (q, *J =* 7.2 Hz, 1H), 2.09 (dhept, *J* = 14.5, 7.0 Hz, 1H), 1.63 (d, *J* = 7.2 Hz, 3H), 1.02 (d, *J* = 6.7 Hz, 3H), 0.83 (d, *J* = 6.8 Hz, 3H) ppm.

**¹³C{¹H} NMR** (176 MHz, CDCl₃): δ = 173.1, 150.9, 140.0, 138.0, 129.5, 127.6, 127.2, 126.0, 122.0 (q, *J* = 255 Hz), 121.5, 85.8, 45.5, 34.5, 18.7, 18.6, 18.2 ppm.

**¹⁹F NMR** (565 MHz, CDCl₃): δ = -57.6 ppm.

**HRMS** (EI): m/z calculated for [C₂₀H₂₁F₃O₃]⁺([M]⁺): 366.1437, measured: 366.1440.

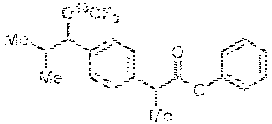

**¹H NMR** (600 MHz, CDCl₃): δ = 7.40 (d, *J* = 8.1 Hz, 1H), 7.34 (t, *J* = 7.9 Hz, 2H), 7.28 (d, *J =* 8.1 Hz, 1H), 7.20 (t, *J* = 7.3 Hz, 1H), 7.00 (t, *J* = 7.6 Hz, 2H), 4.78 (dd, *J* = 7.1, 3.2 Hz, 1H), 3.98 (q, *J* = 7.4 Hz, 1H), 2.10 (dhept, *J* = 13.4, 6.8 Hz, 1H), 1.63 (d, *J* = 7.2 Hz, 2H), 1.03 (d, *J* = 6.7 Hz, 2H), 0.84 (d, *J* = 6.8 Hz, 2H) ppm.

**¹³C{¹H} NMR** (151 MHz, CDCl₃): δ = 173.0, 150.9, 140.0, 138.0, 129.5, 127.6, 127.2, 126.0, 122.0 (q, *J* = 255 Hz), 121.5, 85.8, 45.5, 34.4, 18.7, 18.5, 18.2 ppm.

**¹⁹F NMR** (376 MHz, CDCl₃): δ = -57.8 (d, *J* = 255 Hz) ppm.

**HRMS (EI):** m/z calculated for [C₁₉¹³CH₂₁F₃O₃]⁺ ([M]⁺): 367.1471, measured: 367.1469.

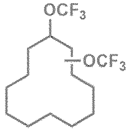

The product was isolated as a mixture of different regioisomers. Therefore, there are different sets of signals in the ¹H, ¹⁹F, and ¹³C NMR, which makes a distinguishment between the different sets very complicated. The signals are given without assignment. Two quartetts resulting from the C-F coupling are shown.

**¹H NMR** (400 MHz, CDCl₃): δ = 4.32 (m, 2 H), 1.86 - 1.55 (m, 8H), 1.54 - 1.25 (m, 12H) ppm. **¹³C{¹H} NMR** (151 MHz, CD₂Cl₂): δ = 124.5, 123.0, 122.0 (q, *J* = 250 Hz),121.8 (q, *J =* 250 Hz), 121.3, 120.3, 118.8, 78.5, 78.5, 78.5, 78.5, 78.0 (q, *J* = 2 Hz), 77.9, 77.9, 77.9, 77.9, 77.9, 77.8, 77.8, 77.5, 77.5, 77.5, 77.5, 30.6, 30.4, 30.3, 30.3, 29.9, 29.9, 29.8, 28.9, 28.8, 28.7, 27.1, 25.7, 24.5, 24.4, 24.3, 23.8, 23.7, 23.4, 21.9, 21.8, 20.7, 20.6, 20.5, 20.5, 19.7, 19.6, 19.1 ppm. **¹⁹F NMR** (376 MHz, CDCl₃): δ = -57.7, -57.8, -57.8, -58.0, -58.0 ppm.

**HRMS (EI):** m/z calculated for [C₁₂H₂₂]⁺ ([M-2OCF₃˙]⁺): 166.1716, measured: 166.1719.

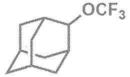

**¹⁹F NMR** (565 MHz, CDCl₃): δ = -57.3 ppm.

**HRMS (EI):** m/z calculated for [C₁₀H₁₅]⁺ ([M-OCF₃˙]⁺): 135.1174, measured: 135.1234.

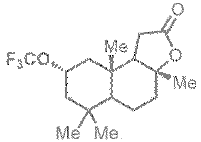

**¹H NMR** (700 MHz, CDCl₃): δ = 4.47 (tt, *J* = 11.5, 4.6 Hz, 1H), 2.45 (dd, *J* = 16.2, 14.7 Hz, 1H), 2.28 (dd, *J =* 16.1, 6.5 Hz, 1H), 2.11 (dt, *J =* 12.0, 3.3 Hz, 1H), 2.02 (dd, *J =* 14.7, 6.5 Hz, 1H), 1.96 - 1.89 (m, 3H), 1.71 (td, *J =* 13.1, 12.6 Hz, 1H), 1.41 - 1.36 (m, 2H), 1.34 (d, *J =* 1.0 Hz, 3H), 1.23 (t, *J =* 11.8 Hz, 1H), 1.12 (dd, *J =* 12.7, 2.8 Hz, 1H), 0.99 (s, 3H), 0.98 (s, 3H), 0.92 (s, 3H) ppm.

**¹³C{¹H} NMR** (176 MHz, CDCl₃): δ = 176.3, 122.0 (q, *J* = 254 Hz), 109.7, 86.1, 73.3, 59.0, 56.1, 48.1, 45.6, 38.7, 37.56, 35.1, 33.4, 29.0, 22.0, 20.5, 16.4 ppm.

**¹⁹F NMR** (376 MHz, CDCl₃): δ = -57.7 ppm.

**HRMS (EI):** m/z calculated for [C₁₇H₂₅F₃O₃]⁺ ([M]⁺): 334.1750, measured: 334.1751.

**X-ray:** Crystals were grown by slow evaporation of a solution of **4f** in CH₂Cl₂ and CyH at ambient temperature.

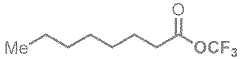

**¹⁹F NMR** (CDCl₃, 376 MHz): δ = -58.6 ppm.

## Claims

1. A photomediated synthesis method for introducing a trifluoroalkoxy moiety into at least one organic compound comprising the following steps:
- Providing at least one organic compound comprising at least one of the following general formulae (I) 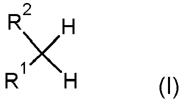
with R¹ being selected from substituted or unsubstituted Aryl or substituted or unsubstituted Heteroaryl,
with R² being selected from H, substituted or unsubstituted Alkyl, substituted or unsubstituted Aryl, substituted or unsubstituted Cycloalkyl and substituted or unsubstituted alkoxycarbonyl, or
- Providing at least one organic compound comprising at least one of the following general formulae (II) 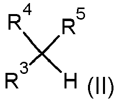
with R³, R⁴, R⁵ being selected from substituted or unsubstituted alkyl, or being a member of a monocyclic, a bicyclic ring system, or polycylic ring system,
wherein R³, R⁴ and R⁵ can be the same or different, and
with R⁵ in addition being selected from H,
wherein R³ and R⁴ can be the same or different, if R⁵ is H, or
- Providing at least one organic compound comprising at least one of the following general formulae (III) 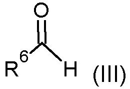 With R⁶ being selected from substituted or unsubstituted aryl or being selected from substituted or unsubstituted alkyl,
- Reacting the at least one organic compound of general formulae (I), (II), (III) with at least one peroxide reagent according to the general formulae (IV)
(OCₘF₂ₘ₊₁)₂
with C being ¹²C or ¹³C,
With m being an integer in the range from 1 to 4, preferably from 1 to 3, most preferably 1 in the presence of at least one organic solvent, and optionally at least one catalyst by irradiating with UV light, and
- Obtaining at least one organic compound comprising at least one of the following general formulae (V), (VI) or (VII) with at least one trifluoroalkoxy moiety 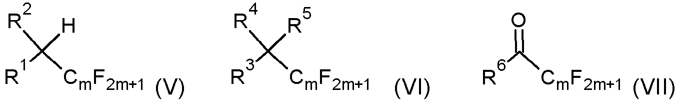 wherein the substituents R¹, R², R³, R⁴, R⁵ and R⁶ have the meanings as described above for general formulae (I), (II) and (III).

2. Method according to claim 1, **characterized in that**
R¹ being selected from substituted or unsubstituted - C₆H₅, preferably - C₆H₅,
- C₆H₄-Hal, preferably -C₆H₄-F, -C₆H₄-CN, -C₆H₄-CF₃,
- C₆H₄-alkyl, preferably -C₆H₄-CH₃, -C₆H₄-C(CH₃)₃,
- C₆H₄-C₆ aryl, preferably -C₆H₄-C₆H₅,
- C₆H₄-C₆H₄-Hal, preferably -C₆H₄-C₆H₄-F, -C₆H₄-C₆H₄-CN, -C₆H₄-C₆H₄-CF₃,
- C₆H₄₋₋C₆H₄-alkyl, preferably -C₆H₄-C₆H₄-CH₃, -C₆H₄-C₆H₄-CH(CH₃)₂ ,-C₆H₄-C₆H₄-C(CH₃)₃,
- C₆H₄-heteroaryl, preferably -C₆H₄-C₅H₄N; or
R¹ being selected from substituted or unsubstituted - C₄-C₅ Heteroaryl, in particular C₄-C₅₋ S, O, N Heteroaryl, such as pyridine, pyrimidine (1,3-dazine), pyridazine (1,2-diazine), pyrazine (1,4 diazine), furan, thiophene-S-oxide, thiopene-S,S-dioxide, and
R² being selected from substituted or unsubstituted C₁-C₆ alkyl, preferably -CH₃, -CH(CH₃)₂), -C(CH₃)₃, substituted or unsubstituted C₁-C₆ alkoxycarbonyl, preferably -(CH₂)ₙOCOR' with n being 1-6, preferably 1-4, R' being C₁-C₆ alkyl, preferably -CH₃, -C₆H₅, -C₆H₄-Hal, such as -C₆H₄-Br, substituted or unsubstituted C₄-C₁₀ cycloalkyl, substituted or unsubstituted C₆ aryl.

3. Method according to claim 1 or 2, **characterized in that**
R¹ 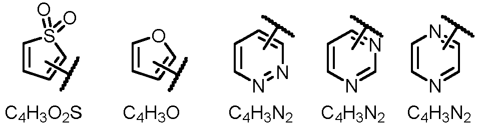
R² 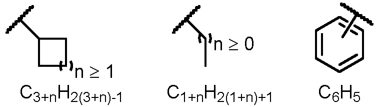
with n being 1-6, preferably 1-4

4. Method according to claim 1, **characterized in that**
R³, R⁴, R⁵ being selected from substituted or unsubstituted C₁-C₆ alkyl or being a member of a monocyclic C₆-C₁₂ cycloalkyl, a -C₆-C₁₂ cycloalkyl fused with lactones, a diamantoide ring systems, such as adamantan, diamantan, wherein R³, R⁴ and R⁵ can be the same or different,and with R₅ in addition being selected from H, wherein R³ and R⁴ can be the same or different, if R⁵ is H.

5. Method according to claim 1, **characterized in that**
R⁶ being selected from substituted or unsubstituted C₆-C₁₂ aryl, preferably - C₆H₅, -C₆H₅-C₆ aryl, preferably -C₆H₅-C₆H₅; -C₆H₄-Hal, preferably -C₆H₄-F, -C₆H₄-CN, - C₆H₄-CF₃, - C₆H₄-alkyl, -C₆H₄-C₁-C₆ alkyl, such as -C₆H₄-CH₃, -C₆H₄-C(CH₃)₃), or being selected from substituted or unsubstituted -C₁-C₁₀ alkyl, such as -C₂-C₈ alkyl, -C₂-C₈ alkyl - aryl, such as -C₇H₁₅, -C₂H₄-C₆ aryl.

6. Method according to one of the preceding claims, **characterized in that** the at least one peroxide reagent according to the general formulae (IV) is selected from one of the following (CF₃O)₂, (C₂F₅O)₂, (C₃F₇O)₂ (linear or branched) or (C₄F₉O)₂ (linear or branched), wherein C is ¹²C or ¹³C, preferably (¹²CF₃O)₂ (known as BTMP) or (¹³CF₃O)₂ (known as ¹³C-BTMP).

7. Method according to one of the preceding claims, **characterized in that** the at least one organic solvent is selected from a group containing dichloromethane (DCM), Acetone, acetonitrile (MeCN), Nitromethane, Dimethyl sulphoxide (DMSO), Ethyl acetate (ETAc) and water (H₂O) or a mixture thereof.

8. Method according to one of the preceding claims, **characterized in that** the at least one catalyst is a tungsten containing salt, in particular a decatungstate containing salt, more in particular an ammonium cation or sodium cation decatungstate containing salt, such as tetrabutylammonium decatungstate (TBADT).

9. Method according to one of the preceding claims, **characterized in that** the reaction is carried out at a temperature between 18°C and 25°C, preferably between 20°C and 23°C, such as room temperature.

10. Method according to one of the claims 1-3, 6-9, **characterized in that** reacting the organic compound general formulae (I) 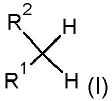 with Bis(trifluoromethyl)-peroxide (BTMP) in the presence of at least one organic solvent, and optionally at least one catalyst, such as tetrabutylammonium decatungstate (TBADT), by irradiating with UV light provides an organic compound general formulae (Va) with one trifluoromethoxy moiety 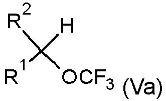
with R¹ being selected from substituted or unsubstituted Aryl or substituted or unsubstituted Heteroaryl,
with R² being selected from H, substituted or unsubstituted Alkyl, substituted or unsubstituted Aryl, substituted or unsubstituted Cycloalkyl and substituted or unsubstituted alkoxycarbonyl.

11. Method according to one of the claims 1, 4, 6-9, **characterized in that** reacting the organic compound of general formulae (II) 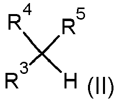 with Bis(trifluoromethyl)-peroxide (BTMP) in the presence of at least one organic solvent, and optionally at least one catalyst, such as tetrabutylammonium decatungstate (TBADT), by irradiating with UV light provides an organic compound general formulae (Vla) with one trifluoromethoxy moiety 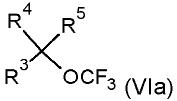
with R³, R⁴, R⁵ being selected from substituted or unsubstituted alkyl, or being a member of a monocyclic, a bicyclic ring system, or polycylic ring system, and
with R₅ in addition being selected from H, or
wherein R³ and R⁴ can be the same or different, if R⁵ is H.

12. Method according to one of the claims 1, 5-9, **characterized in that** reacting the organic compound of general formulae (III) 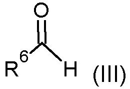 with Bis(trifluoromethyl)-peroxide (BTMP) in the presence of at least one organic solvent, and optionally at least one catalyst, such as tetrabutylammonium decatungstate (TBADT), by irradiating with UV light provides an organic compound general formulae (Vlla) with one trifluoromethoxy moiety 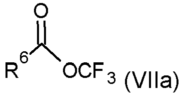 with R⁶ being selected from substituted or unsubstituted aryl or being selected from substituted or unsubstituted alkyl.

13. Compound obtainable in a method according to one of the claims 1-3, 6-9 **characterized by** the following structures:

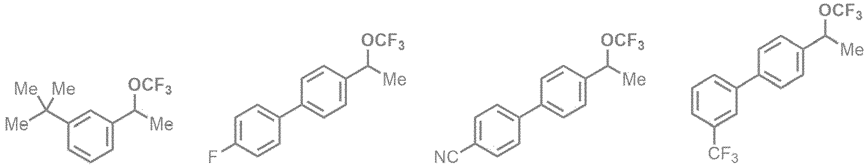

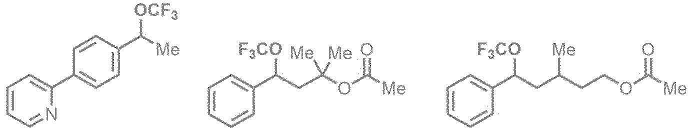

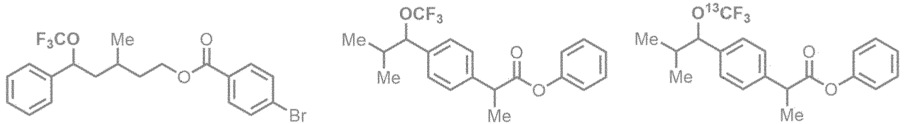

14. Compound obtainable in a method according to one of the claims 1, 4, 6-9 **characterized by** the following structures:

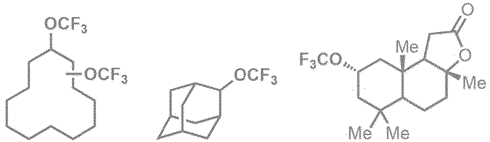

15. Compound obtainable in a method according to one of the claims 1, 5-9 **characterized by** the following structures:
